# EUROPEAN PATENT APPLICATION

(11) **EP 3 808 383 A2**
(43) Date of publication of application: **21.04.2021**
(21) Application number: 20201611.9
(22) Date of filing: 13.10.2020
(51) Int. Cl.: A61L 2/24, A61L 2/07, G07F 7/02, G07F 7/06

(54) **TAG READING TRIGGERED BOTTLE STERILISATION AND REFILL UNIT AND PROCESS**

(30) Priority: 14.10.2019 GB 201914804
(71) Applicant: IRB, d.o.o, 2352 Selnica ob Dravi (SI)
(72) Inventor: Bonetto, Luca, 2310 Slovenska Bistrica (SI)
(74) Representative: Koplin, Moritz

(57) **Abstract**

An apparatus (100) for sterilizing and refilling a bottle (10). The apparatus includes a tag reader (100a), a sterilizer (119), a fluid supply (124) and a controller (116). The controller is configured to select a sterilization procedure and/or a refill-fluid based on information associated with a bottle tag.

## Description

### Technical Field

The present disclosure relates to sterilizing and refilling a bottle. In particular, the present disclosure relates to an apparatus for sterilizing and refilling a bottle, wherein sterilization and refill of the bottle may also be based on information associated with a bottle tag.

### Background Art

EP2625134 A1 teaches a vending machine for dispensing, receiving, cleaning and/or filling containers. The machine comprises container receiving means for receiving a container, container cleaning means for cleaning the container, container dispensing means for dispensing the container, and container filling means for filling the container.

### Summary of Invention

The present invention provides an apparatus for sterilizing and refilling a bottle which comprises a tag reader, a sterilizer, a fluid supply, and a controller, wherein the controller is configured to select a sterilization procedure and/or a refill-fluid based on information associated with a bottle tag.

In this regard, the term "bottle", as used throughout the description and the claims, particularly refers to a container made of an impermeable material (stainless steel, glass,
plastic, aluminum etc.) that can be sealed with a closure and that may or may not be insulated. Moreover, the term "sterilizing", as used throughout the description and the claims, particularly refers to reducing the number of microorganisms within the bottle. For example, the sterilizer may be configured for moist heat sterilization.

Furthermore, the term "tag reader", as used throughout the description and the claims, particularly refers to a sensor for detecting data identifying the bottle. For example, the bottle may be provided with a radio frequency identification, RFID, tag, which allows the (wireless) determination of the (unique) serial number of the bottle. The RFID tag may be embedded into the bottom or the wall of the bottle to avoid separation of the tag from the bottle. In another example, the tag may be a visual tag such as a bar code (e.g., a matrix bar code) that allows to (optically) determine the (unique) serial number of the bottle.

In addition, the term "fluid supply", as used throughout the description and the claims, shall encompass one or more (connected and/or selectable) containers containing a fluid (such as water) but also a tap water supply. Finally, the term "controller", as used throughout the description and the claims, particularly refers to an electronic device comprising a processor and a memory which persistently stores a set of instructions which, when carried-out by the processor, govern the operation of the apparatus.

The controller may be configured to retrieve the information from a server.

For example, the server may be a remote (physical or virtual) entity that is connected to
the controller through a cellular network and/or a wide-area network. The server may store the user account of the owner of the bottle. The user account may be associated with a credit or a payment option through which the user can be billed (for usage of the apparatus). For example, the user may add credit to the user account by making a payment or by selecting a payment option (e.g., credit card) which allows direct debiting.

In order to prevent that the throughput of the apparatus is decreased due to users who do not have enough credit (or another valid payment option) but nevertheless (unsuccessfully) try to use the apparatus, the controller may be configured to allow inserting the bottle into a sterilization and/or refill chamber only if there is an account associated with the bottle tag that has enough credit.

The user account may also comprise preferences as regards the refill-fluid. Theses preferences may be updated ("manually") by the user or automatically by an application which, for instance, tracks user habits, the surroundings (e.g., the weather), and/or user activities (e.g., sports). The preferences may indicate how much carbon dioxide, which vitamins and/or which flavor may be added to the water.

I.e., the controller may be configured to select an amount of gas and/or ingredients to be added to water supplied by the fluid supply, to produce the refill-fluid, based on the information. Moreover, the controller may be configured to select a temperature of the refill-fluid based on the information. Furthermore, the controller may be configured to allow modifying and/or supplementing the information based on user input received from a user interface of the apparatus

For example, an owner of the bottle may use public transportation and decide to get a refill. The person may then log into her/his account (e.g., via an app of her/his smartphone) check whether enough credit or a suitable payment option is selected and update her/his preferences as regards the refill-fluid (carbonation, vitamins, flavor, temperature, etc.). When reaching the apparatus, the owner may place her/his bottle close to the tag reader. The tag reader may send the data from the bottle tag to the controller, which allows identifying the user account by querying the server. Having identified the user account and checked the balance/payment option, the apparatus may open a port. Sterilizing and refilling may then start once the owner of the bottle has inserted the bottle into the port and closed the port using the preferences/history associated with the user account, unless the user decides to override the preselected parameters via a user interface of the apparatus. As such, user interaction with the apparatus and hence the time required for getting a refill is minimized.

The apparatus may further comprise a transmitter connected to the controller for establishing a communication channel with a mobile device and initiating a procedure for adding credit to the account. For example, if the bottle is rejected for lack of credit, credit may be instantly added to the account by paying via mobile phone.

The apparatus may be configured to inspect the bottle and replace the bottle if the bottle is damaged.

For example, if the bottle is broken or deformed, the bottle may be replaced, and the user account may be updated to reflect the replacement.

The apparatus may be configured to replace the bottle with a larger or smaller bottle if the information associated with the bottle tag includes a corresponding request.

For instance, a user may opt for a replacement depending on activities of the user. Thus, the user may select a relatively small bottle in the morning when going to work and a relatively large bottle in the evening when going to the gym.

The apparatus may be configured to replace the bottle with a bottle having another shape and/or color, if the information associated with the bottle tag includes a corresponding request.

For example, a user may opt for a replacement depending on activities of the user. Thus, the user may select a black bottle in the morning when going to work and a yellow/red/green/blue/pink bottle in the evening when going to the gym.

The apparatus may have an input port and an output port which is separate from the input port and the apparatus may be configured to refill a first bottle while sterilizing a second bottle.

This may further increase the bottle throughput.

A system may comprise two (or more) apparatus and a central server configured to receive data on a bottle tag from the first or the second apparatus and transmit the information associated with the bottle tag to said apparatus.

A system may comprise the apparatus, the server and mobile device, wherein the mobile device is configured to allow choosing a sterilization procedure and/or a refill-fluid and to associate the chosen sterilization procedure and/or refill-fluid with the bottle tag and, wherein the server is configured to forward information on the sterilization procedure and/or the refill-fluid to the controller in response to a request of the controller for information associated with the bottle tag.

The present invention further provides a method for sterilizing and refilling a bottle which comprises transmitting, by a mobile device, information on a sterilization procedure and/or a refill-fluid to a server, reading, by an apparatus for sterilizing and refilling a bottle, a bottle tag, requesting, by the apparatus, information on the sterilization procedure and/or the refill-fluid associated with the bottle tag from the server, transmitting, by the server, the information on the sterilization procedure and/or the refill-fluid to the apparatus for sterilizing and refilling the bottle, and sterilizing and/or refilling the bottle, by the apparatus, in accordance with the information.

The sterilization procedure and/or the refill-fluid may be selected manually by a user of the mobile device or determined automatically based on user data collected by the mobile device. Furthermore, the apparatus may only allow certain procedures or require the acceptance of a disclaimer in case of discrepancy between the information related to the bottle and the sterilization procedure selected by the user. If, for instance, the server has no records of the bottle being sterilized for a given period and the user selects a quick wash procedure, the apparatus may ask the user to acknowledge (a disclaimer stating) that the apparatus may not guarantee a sufficient hygiene level based on the user's specific request or to select a more intense sterilization procedure.

The user data may include information on a workout performed by the user.

It will be appreciated that the features and attendant advantages of the disclosed method may be realized by the disclosed apparatus and vice versa. Moreover, it is noted that throughout the description, features in brackets are to be regarded as optional.

### Brief Description of Drawings

The foregoing aspects and many of the attendant advantages will become more readily appreciated as the same becomes better understood by reference to the following description of embodiments, when taken in conjunction with the accompanying drawings, wherein like reference numerals refer to like parts throughout the various views, unless otherwise specified.
Fig. 1 schematically illustrates the usage of the apparatus in an exemplary environment;
Fig. 2 shows a possible modification of the apparatus of Fig. 1;
Fig. 3 illustrates the communication between the entities shown in Fig. 1; and
Fig. 4 shows a flowchart illustrating the operation of the apparatus of Fig. 1.

Notably, the drawings are not drawn to scale and unless otherwise indicated, they are merely intended to conceptually illustrate the structures and procedures described herein.

### Description of Embodiments

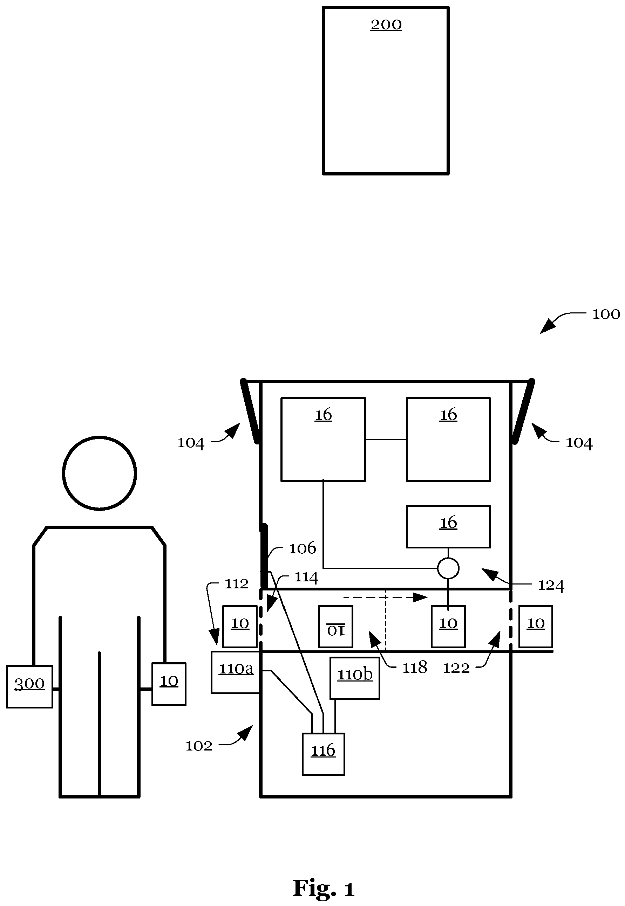

Fig. 1 shows an apparatus 100 for sterilizing and refilling bottles 10. The apparatus 100 may comprise a stainless steel- or aluminium-supported structure with external walls 102 made from plastic (e.g., fibreglass and/or polycarbonate). The casing of the apparatus 100 may be provided with an intrusion detection system to prevent theft and demolition, as the apparatus 100 maybe located in high-traffic areas. Moreover, the apparatus 100 may be provided with (colour) monitors 104 (to display ads) and a user interface 106 (e.g., a recessed colour touch screen) to display messages and receive user input.

The apparatus 100 may further comprise an external tag reader 110a for determining an identification, ID, of a bottle 10 which is placed in close proximity of the external tag reader 110a. For example, the external tag reader 110a may be embedded into a support 112 (or stand which is outside the apparatus 100) onto which the bottle 10 can be placed. The support 112 may be provided with a recess or a cavity that matches the size of the bottom of the bottle 10 to facilitate positioning of the bottle 10 relative to the external tag reader 110a. The external tag reader 110a may be embedded into or positioned behind an external wall 102 (if the tag reader 110a can read through the wall 102). In both cases, the external tag reader 110a may be placed at a position where the user may operate the user interface 106 while holding or immediately after positioning the bottle 10 within a detection area of the external tag reader 110a.

The ID determined by the external tag reader 110 may be used to decide whether the bottle 10 qualifies for (instant) sterilization and refill. For instance, if a bottle 10 cannot be identified, the input port 114 may be kept close to prevent the owner of the bottle 10 from inserting the bottle 10 into the apparatus 100. Alternatively, the input port 114 may (always) be open but require attaching the bottle 10 to a feeder (e.g., a swivelling arm). In that case, if a non-tagged bottle 10 or another item is inserted into the apparatus 100, the non-tagged bottle 10 or the other item may not match the feeder (e.g., the item may slip out of the feeder), hence making it difficult to "trick" the apparatus 100.

If the bottle that is inserted is not fully empty (which may be detected based on a strain gauge, as the bottle's 10 weight may not conform with the bottle's 10 specification), the apparatus 100 may refuse to sterilize and/or refill the bottle 10. Alternatively, the apparatus 100 may be provided with a sink and the fluid in the bottle 10 may be poured into the sink before sterilization and/or refill. The apparatus 100 may also use the feeder (or another mechanism, e.g., a trapdoor) to remove any non-tagged and/or full bottle 10 from the apparatus 100.

If the bottle 10 can be identified, a controller 116 of the apparatus 100 may query a server 200 whether the ID determined by the tag reader 110 is associated with a user account. To this end, the apparatus 100 may be provided with a (4G) modem that enables the apparatus 100 to communicate with the server 200 over the internet. In case that the ID is not associated with a user account, no further operations may be allowed. If the ID is associated with a user account, it may be determined whether the user account has enough credit. If the user account has insufficient funds, the owner of the bottle 10 may be asked to pay for the sterilization and refill over a top-up contactless payment surface.

In case of a low balance, the apparatus 100 may show a warning (via the user interface 106). Once the user has paid, or if it has been determined that enough credit is associated with the user account, the user may select a level of carbonation, vitamins, temperature, and/or a flavor through the user interface or, if such information is available through the user account, the user preferences may be provided from the server 200 to the controller 116. The same may apply to the sterilization process which may be selected by the user through the user interface or which may be determined as part of the user preferences/history or based on the length of the interval from the last documented sterilization procedure. Notably, user accounts stored locally on a storage within the apparatus (and accessible to the controller 116) maybe synchronized with user accounts stored on the server 220 (or within a cloud) to avoid communication delays.

The user account may allow to keep track of the last time, the bottle 10 was sterilized and filled at the apparatus 100 (or another similar apparatus 100). In case that the interval is longer than a predefined period (e.g., 3 days) the apparatus 100 may refuse to proceed with the cycle unless the user agrees to a disclaimer regarding the sterility of the bottle 10 or approves the additional cost of an extensive sterilization procedure. Moreover, the apparatus 100 maybe provided with an internal tag reader 110b (instead of, or in addition to the external tag reader 110a). If the internal tag reader 100b is in addition to the external tag reader 110a, it can be determined whether the bottle 10 inserted into the apparatus 100 is the same bottle 10 as the bottle 10 which has been detected by the external tag reader 110b to prevent misuse of any kind.

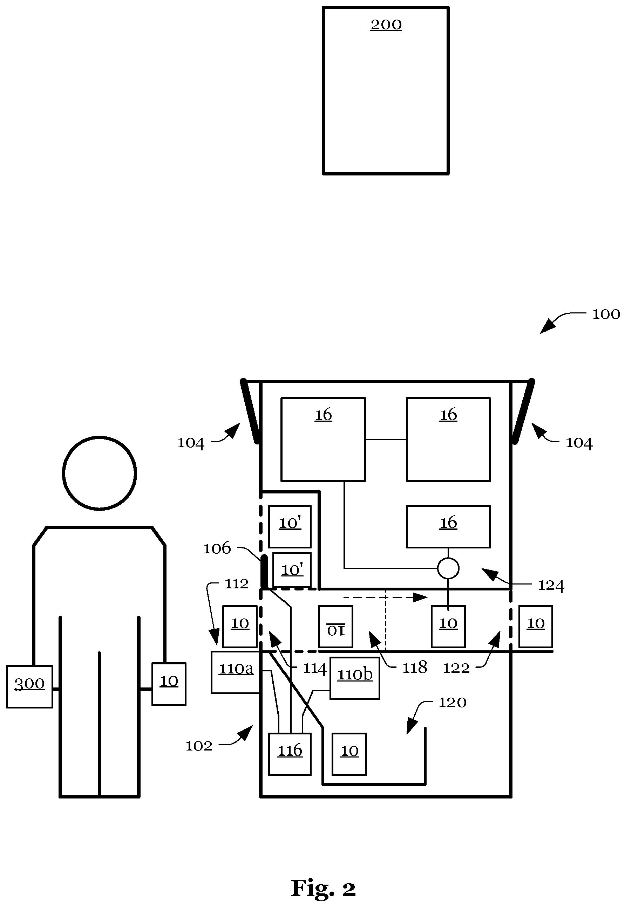

Once the length and/or heat of the sterilization procedure is determined, the input port 114 may be opened such that the user can insert the bottle 10 into the apparatus 100, or in case the input port 114 is always open, the process may start right away. Within the apparatus 100, the bottle 10 may be inspected and replaced with another bottle 10 (if damaged) as schematically illustrated in Fig. 2. Damaged bottles 10 may be collected in a container 120 within the apparatus 100. Moreover, if requested by the user, a bottle 10 may be replaced with a bottle 10' having another size or color. To this end, a limited number of replacement bottles 10' may be stored within the apparatus 100.

Once the bottle 10 is positioned inside the apparatus 100, the feeder may move the bottle 10 into the sterilizer 118 (which may be visible through a pane), where the bottle 10 may be turned upside down. Within the sterilizer 118, a steam nozzle may be inserted inside the bottle 10 and proceed to clean it out. In addition (depending on the user preferences/selection) a UV-source may be used to (further) sterilize the outside (and inside) of the bottle 10. Moreover, the sterilizer 118 may allow sterilizing the bottle's 10 closure (cap). In case of longer intervals since the last usage of the bottle or if specifically requested by the user, a high-pressure water jet or other physical procedure for the removal of solid or gelified residues may also be applied. After sterilization, the bottle 10 may be swiveled back and moved to the filling station 122 where it may be connected to the fluid supply 124. The filling station 122 may be provided with a protective ABS shield.

Regarding the fluid supply 124, the apparatus 100 may be provided with one or more racks for multiple fluid containers (e.g., 201 bottles or stainless steel kegs, such as sanke kegs) which are connected and/or selectable by the controller. The racks may be mounted on wheels in order to facilitate exchanging the containers. The fluid (e.g. water) may be collected from the containers in an individual rack at a time and distributed between (two) different processing units, which may or may not comprise intermediate holding tanks. One processing unit may be a cooler, capable of reaching low temperatures (e.g., 5°C), by means of a compressor or otherwise, and another processing unit may be a boiler, capable of reaching higher temperatures (above 30°C).

The fluid supply 124 may further comprise an automatic dosing system which provides for an accurate dosage of predetermined amounts of water (e.g. 250ml, 370ml, 500ml) by mixing amounts from the two different processing units. The fluid supply 124 may be connected to a CO2 addition system which may be suitable for different levels of carbonation (e.g., none, light, medium, and heavy). In order to give the fluid a flavour or to add certain ingredients, a syrup may be added to the fluid (e.g., liquid Magnesium, liquid stevia, multivitamin syrup, liquid Vitamin D, cranberry extract, mixed electrolytes for sports, etc.).

The user's filling history, as well as her/his habits regarding favourite drinks may be stored by an application which runs on a mobile device 300 or as part of a user account on the server 200. The application may allow the user to register her/his bottles 10 by touching an NFC sensor on the mobile device 300 against the RFID tag on the bottles 10 and potentially to produce a profile for each bottle 10 she/he owns. In case the registration is not possible via an NFC tap, a unique code may be manually entered or a QR code may be scanned.

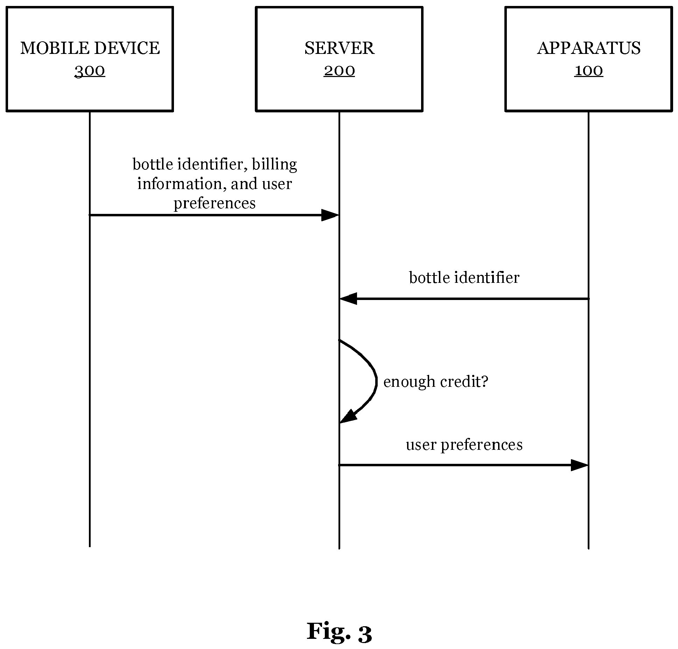

As shown in Fig. 3, an application on the mobile device 300 may transmit a bottle identifier, billing information and user preferences to the server 200 which stores the information in association with a user account. Alternatively, the information may be stored on the mobile device 300 and transmitted directly to the apparatus 100.

The application may interface with a health tracking application in order to reward the user every time she/he purchases a drink and record this automatically with the health tracking application. For example, the user may insert her/his bottle 10 in the apparatus 100 and buy a vitamin cocktail. The application may receive the notification and reward the user by complimenting her/him, confirming the purchase and leaving an open notification on the notification screen, requesting the user to discard or register the water and vitamins consumption. Upon selection of the "register" option within the notification, a health tracking app may record the drink of water and vitamins.

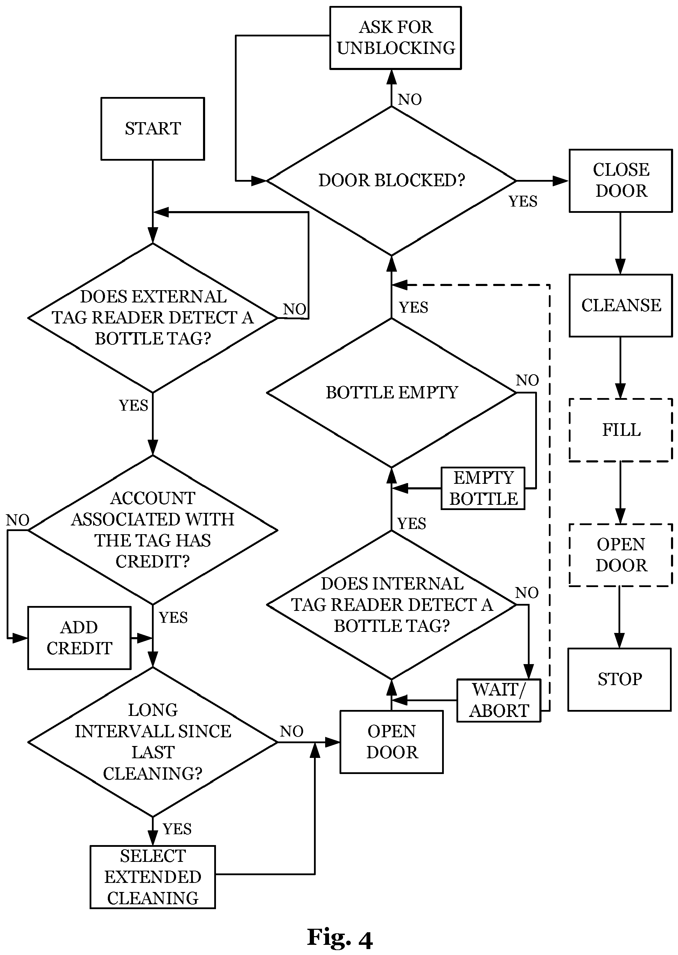

Fig. 4 shows a flowchart illustrating the operation of the apparatus 100 of Fig. 1. When the apparatus 100 is in standby, the input port 114 (door) is closed and the external tag reader 110a is operated to detect a bottle tag. When a bottle tag is detected, it may be checked whether the account associated with the bottle tag has enough credit. If the account has not enough credit, the owner of the bottle 10 may be prompted to add credit to the account. For example, the owner may add credit to the account by using the application on her/his mobile device 300 or the user interface 106. Notably, the user interface 106 may also be used to establish a wireless connection between the mobile device 300 and the apparatus 100 to add credit to the account using a payment process involving the mobile device 300.

Furthermore, the cleaning history of the bottle 10 may be checked. In case that an interval since the most recent cleaning is longer than a threshold, an extended cleaning operation may be automatically selected. The extended cleaning operation may differ from a regular cleaning operation in regard to the duration and/or the temperature of the procedure (and/or the application of UV light). Moreover, the duration/temperature may be increased gradually depending on the interval. In another embodiment, the owner of the bottle 10 may be prompted to agree to the extended cleaning operation or confirm that the bottle is clean. For example, the owner may have washed the bottle 10 herself/himself in a dishwasher.

Once the cleaning procedure is set, the door may be opened which allows the owner to insert the bottle 10 into the apparatus 100. To avoid that another bottle than the bottle 10 which has been initially detected undergoes sterilization and refill, the bottle tag may be detected by the internal tag reader 110b. If no bottle 10 is detected after a certain period, the apparatus 10 may abort the session, close the door, cleanse the interior (for safety reasons) and return to standby. In another embodiment, if a bottle without a bottle tag is detected, the bottle may be automatically removed, or the owner of the bottle may be prompted to remove the bottle. After that, the interior of the apparatus 100 maybe cleansed (for safety reasons) and the apparatus 100 may return to standby.

If the internal tag reader 110b detects a bottle 10, the feeder may check whether the bottle 10 is empty. If the bottle 10 is not empty, the feeder may try to empty the bottle 10 or prompt the owner to remove/empty the bottle 10. In another embodiment, if a non-empty bottle 10 is detected, the bottle 10 may be automatically removed, the apparatus 10 may abort the session, cleanse the interior (for safety reasons) and return to standby. In case that the feeder confirms that the bottle 10 is empty (or has been emptied), the apparatus 100 may check whether the door is blocked and if the door is not blocked, close the door. In case that the door is blocked, the apparatus 100 may inform the user of the situation and wait until the door is no longer blocked. In another embodiment, the apparatus 100 may use the feeder to (gently) remove unwanted items from the input port 114.

After closing the door, the apparatus 100 may perform the cleansing and filling. After cleansing and filling, the apparatus may open the door and prompt the user to remove the bottle 10. In another embodiment, the apparatus 100 may remove the cleansed and refilled bottle 10 automatically. Moreover, in case that the interior is only cleansed for safety reasons, filling and opening the door may be skipped and the apparatus 100 may directly return to standby.

During longer standby periods (e.g. during the night), the apparatus may be programmed to carry out a self-sterilisation procedure, through which the components of the apparatus that are exposed to non-refrigerated liquids, to air-liquid mixes and/or to human interaction are thoroughly cleaned and sterilised. This may be carried out by moist heat. The fluid to produce warm drinks (e.g., tea) and steam for sterilizing may come from a single pressurised boiler (such as those installed in coffee machines).

### Reference signs list

10 bottle
100 apparatus
102 external wall
104 monitor
106 user interface
110a tag reader
110b tag reader
112 support
114 input port
116 controller
118 sterilizer
120 container
122 filling station
124 fluid supply
200 server
300 mobile device

## Claims

1. An apparatus for sterilizing and refilling a bottle, the apparatus comprising:
a tag reader;
a sterilizer;
a fluid supply; and
a controller;
wherein the controller is configured to select a sterilization procedure based on information associated with a bottle tag;
wherein the information comprises a usage pattern of the bottle.

2. The apparatus of claim 1, wherein the controller is configured to retrieve the information from a server.

3. The apparatus of any one of claim 1 or 2, wherein the controller is configured to allow modifying and/or supplementing the information based on user input received from a user interface of the apparatus.

4. The apparatus of any one of claims 1 to 3, wherein the controller is configured to select a refill fluid based on the information acquired.

5. The apparatus of any of claims 1 to 4, wherein one of multiple fluids available for selection comprises bottled water stored in pressurised containers in the apparatus.

6. The apparatus of any one of claims 1 to 5, wherein the controller is configured to select a temperature of the refill-fluid and/or an amount of gas and/or ingredients to be added to water supplied by the fluid supply to produce the refill-fluid, based on the information.

7. The apparatus of any one of claims 1 to 6, wherein the sterilizer is configured for moist heat sterilization.

8. The apparatus of any one of claims 1 to 7, wherein the controller is configured to allow inserting the bottle into a sterilization and/or refill chamber only if there is an account associated with the bottle tag that has sufficient credit.

9. The apparatus of claim 8, further comprising:
a transmitter connected to the controller for establishing a communication channel with a mobile device and initiating a procedure for adding credit to the account.

10. The apparatus of any one of claims 1 to 9,
wherein the apparatus is configured to inspect the bottle and replace the bottle if the bottle is damaged and/or
wherein the apparatus is configured to replace the bottle with a larger or smaller bottle if the information associated with the bottle tag includes a corresponding request or if such request is received at a/the user interface of the apparatus.

11. The apparatus of any one of claims 1 to 10, wherein the apparatus is configured to replace the bottle with a bottle having another shape and/or color, if the information associated with the bottle tag includes a corresponding request or if a corresponding request is received at a/the user interface of the apparatus.

12. The apparatus of any one of claims 1 to 11, wherein the apparatus has an input port and an output port which is separate from the input port and wherein the apparatus is configured to refill a first bottle while sterilizing a second bottle.

13. A system, comprising:
a first apparatus for sterilizing and refilling a bottle according to any one of claims 1 to 12;
a second apparatus for sterilizing and refilling a bottle according to any one of claims 1 to 12; and
a central server configured to receive data on a bottle tag from the first or the second apparatus and transmit the information associated with the bottle tag to said apparatus.

14. A system, comprising:
an apparatus for sterilizing and refilling a bottle according to any one of claims 1 to 12;
a/the server; and
a/the mobile device;
wherein the mobile device is configured to allow choosing a sterilization procedure and/or a refill-fluid and to associate the chosen sterilization procedure and/or refill-fluid with the bottle tag; and
wherein the server is configured to forward information on the sterilization procedure and/or the refill-fluid to the controller in response to a request of the controller for information associated with the bottle tag.

15. A method for sterilizing and refilling a bottle, the method comprising:
transmitting, by a mobile device, information on a sterilization procedure and/or a refill-fluid to a server;
reading, by an apparatus for sterilizing and refilling a bottle, a bottle tag;
requesting, by the apparatus, information on the sterilization procedure r;
transmitting, by the server, the information on the sterilization procedure; and
sterilizing and/or refilling the bottle, by the apparatus, in accordance with the information;
wherein the sterilization procedure is determined automatically by the apparatus based on a usage pattern of the bottle.
